(19)

Europäisches
Patentamt
European
Patent Office
Office européen
des brevets

(11) **EP 2 098 236 A1**

(12) **EUROPEAN PATENT APPLICATION**

(43) Date of publication:
**09.09.2009 Bulletin 2009/37**

(51) Int Cl.:
*A61K 31/52* (2006.01)      *A61P 35/02* (2006.01)
*A61P 1/04* (2006.01)       *A61P 29/00* (2006.01)

(21) Application number: **08003920.9**

(22) Date of filing: **03.03.2008**

<table>
<tr><td>

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Medizinische Hochschule Hannover 30625 Hannover (DE)**

</td><td>

(72) Inventor: **Stanulla, Martin.**
**30177 Hannover (DE)**

(74) Representative: **Kröncke, Rolf et al**
**Gramm, Lins & Partner**
**Freundallee 13 a**
**30173 Hannover (DE)**

</td></tr>
</table>

(54) **Cytostatic compositions**

(57)  The present invention relates to new cytostatic composition comprising sub-cytostatic dosages of a first purine analog or a pharmaceutically acceptable salt thereof and a sub-cytostatic dosage of a second purine analog which is different to the first purine analog or a pharmaceutically acceptable salt thereof. The invention relates to the use of said cytostatic composition for treating cancer, autoimmune diseases and inflammatory bowel disease.

Figure 2.

Dose (µM)

EP 2 098 236 A1

**Description**

The present invention relates to cytostatic compositions, and in particular, the present invention relates to the co-administration of sub-cytostatic dosages of two purine analogs for an improved therapeutic efficacy.

**Background of the invention**

Antimetabolites are structural analogs of naturally occurring substances interfering with the synthesis of nucleic acids. For example, 6-thioguanine is a guanine analog and may be incorporated into RNA and DNA substituting guanine nucleotides. Said anti-metabolites may act on different levels comprising competing for binding sites of enzymes, incorporation into nucleic acid molecules or interfering with DNA synthesis of purines and guanine nucleotides. Antimetabolites inhibit growth of fast proliferating cells of an individual, e.g. cancer cells, bone marrow cells or cells present in the gastrointestinal tract. Various categories of antimetabolite exists, one of the main type of anti-metabolites are purine analogs.

Two of the most prominent purine analogs used in cancer therapy are the thiopurines 6-mercaptopurine and 6-thioguanine. The principle use lies in the treatment of acute leukemias, lymphomas, rheumatoid arthritis, and inflammatory bowel disease, such as Crohn's Disease and ulcerative colitis, respectively.

For example, 6-thioguanine is used during early and/or late intensification therapy of childhood acute lymphoblastic leukemia (ALL) while 6-mercaptopurine is mainly used at a different time point of therapy, namely during maintenance treatment of ALL. Azathioprine is another member of the antimetabolites and represents a prodrug of 6-mercaptopurine in general. Azathioprine is metabolized in the liver to form 6-mercaptopurine. Azathioprine is widely used as an immunosuppressant for the treatment of conditions such as inflammatory bowel disease, autoimmune conditions and following organ transplantation.

Over 50 years ago, 6-mercaptopurine was approved as a drug for treatment of ALL in children. The thiopurines are pro-drugs and have to be metabolized in order to exert their cytotoxic action. Azathioprine is non-enzymatically reduced to 6-mercaptopurine and an imidazole group. Both 6-mercaptopurine and 6-thioguanine undergo extensive metabolism before excerting cytotoxicity following incorporation into DNA as thioguanine nucleotides (TGNs) or in the case of 6-mercaptopurine, inhibition of de novo purine synthesis.

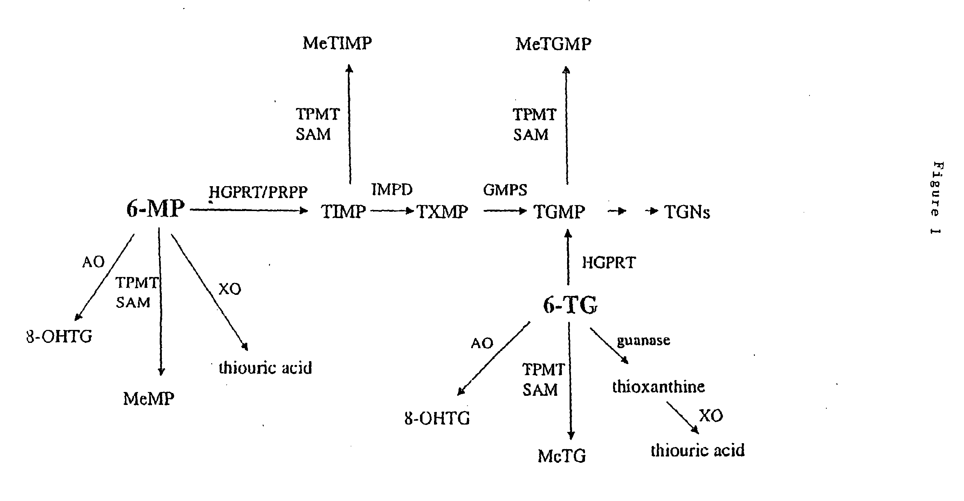

Figure 1 shows the metabolic pathway of 6-mercaptopurine and 6-thioguanine, respectively. As can be seen from figure 1 the main enzymes competing for the initial metabolism of 6-mercaptopurine and 6-thioguanine are hypoxanthine-guanine-phosphoribosyltransferase (HGPRT), thiopurine S-methyltransferase (TPMT), aldehyde oxidase (AO), and xanthine oxidase (XO). Both XO and AO produce metabolites believed to have little or no cytotoxic action. In the case of 6-thioguanine, XO can only metabolize the drug after prior conversion by guanase. As the level of XO activity in the bone marrow is very low, this is unlikely to affect the activity of the thiopurine drugs at their main site of action. TPMT varies in activity and is the main enzyme responsible for the metabolism of these drugs. 6-thioguanine forms thioguanisine monophosphate (TGMP) after metabolism by HGPRT. TGMP is further metabolized to form di- and triphosphate TGNs which subsequently become incorporated into RNA and DNA. 6-mercaptopurine has to be converted first into the 6-thioinosine-5'-monophosphate intermediate. Said intermediate may act as a strong inhibitor of the de novo purine synthesis which is believed to exert a significant contribution to the cytotoxic action of 6-mercaptopurine. Said intermediate may also be metabolised by the TPMT enzyme.

An effect of TPMT activity levels on cytotoxicity of these drugs was reported already in 1987 by van Lohn et al., Journal of Pharmacology and Experimental Therapeutics, 1987, 242: 21-26.

For example, in childhood ALL, thiopurines play an important part of the therapeutic regimen. For no other than historical reasons, 6-mercaptopurine is used in ALL while 6-thioguanine is mainly used in acute myeloid leukemia (AML) or relapsed ALL. Several contemporary treatment protocol for childhood ALL apply consecutive cycles of either 6-mercaptopurine or 6-thioguanine starting as early as during induction consolidation treatment and continue administration during maintenance therapy for up to 36 months after diagnosis. As prodrugs thiopurines require bioactivation by a multiple step pathway to form TGNs, as described above. After induction of remission, thiopurines are used almost throughout the entire therapy. The most extensive application of thiopurines occurs during the maintenance phase. Maintenance treatment aims at a further stabilization of remission by suppressing the re-emergence of a drug resistant clone through consistently reducing the pool of residual leukemic cells. The current standard of maintenance therapy consists of up to 2 to 3 years of treatment with daily oral 6-mercaptopurine and weekly oral methotrexate. The combination of 6-mercaptopurine with methotrexate acts synergistically as methotrexate inhibits purine de novo synthesis leading to a higher intracellular availability and increased incorporation of phosphorylated thiopurines in DNA and RNA.

Until today there is an ongoing debate on the better thiopurine and various published randomized studies exist comparing the toxicity and efficacy of 6-thioguanine with 6-mercaptopurine in interim maintenance and maintenance therapy of childhood ALL. There are three published randomized studies comparing the toxicity and efficacy of 6-thioguanine with 6-mercaptopurine in interim maintenance and maintenance therapy of childhood ALL. The first published trial randomized 474 patients with childhood ALL to either 6-thioguanine or 6-mercaptopurine in maintenance and was conducted by the German COALL study group (Harms et al. Blood. 2003, 102: 2736-2740). The COALL-92 trial results show no difference in the primary outcomes, but observed a tendency of higher central nervous system (CNS) relapse (3.4% vs. 0.8%, p = 0.053) and prolonged myelosuppression with marked thrombocytopenia in the 6-thioguanine treatment arm. In COALL-92, the incidence of thrombocytopenia ($< 100 \times 10^9$/l) without evidence of leukocytopenia ($<1 \times 10^9$/l) was 7.5-fold higher in the 6-thioguanine compared to the 6-mercaptopurine arm. Treatment interruptions occurred at a 1.7-fold higher rate in the 6-thioguanine arm (4.7% of maintenance treatment weeks vs. 2.8% for 6-mercaptopurine). A recent British trial by Vora and colleagues (Lancet. 2006, 368: 1339-1348), UK MRC ALL 97, randomized 1498 children to receive either 6-thioguanine or 6-mercaptopurine. After a median follow-up of six years, no differences in event-free survival were detected between the two treatment arms. A large reduction of isolated disease recurrence in the CNS by 6-thioguanine (odds ratio 0.53, 95% confidence interval 0.30 - 0.92) was offset by an increased risk of death in remission (odds ratio 2.22, 95% confidence interval 1.20 - 4.14), mainly due to infections. Strikingly, 11% of patients in the 6-thioguanine arm compared to less than 2% in the 6-mercaptopurine arm developed non-fatal hepatic toxicity with features of veno-occlusive disease (VOD) characterised by symptoms including tender hepatomegaly, hyperbilirubinaemia with elevated aminotransferases, thrombocytopenia out of proportion to neutropenia, and portal hypertension. In the majority of 6-thioguanine recipients, these symptoms were observed during maintenance or interim maintenance (85%). Of interest, in patients randomised to 6-mercaptopurine, hepatic toxicity was associated with intensification elements in which both treatment arms received exclusively 6-thioguanine. A third, not yet completely published study (Stork et al. Blood. 2002, 100: 156a) randomising 6-thioguanine and 6-mercaptopurine in childhood ALL is the US trial CCG-1952 including more than 2000 patients with lower-risk features at diagnosis (NCI standard-risk ALL). This trial suggests for the 6-thioguanine arm a significantly better event-free survival (85.1% vs. 77.1 % at 5 years, p = 0.02), less CNS and also bone marrow and testicular relapses without differences in remission deaths between the arms. Of importance, 6-thioguanine administration led to hepatic VOD-like symptoms in approximately 20% of patients. Looking at 6-thioguanine-related efficacy and toxicity in the three trials, it is obvious that the CCG-1952 trial shows the greatest benefit for the 6-thioguanine arm at the highest rate of hepatotoxic side-effects. This trial also used the highest starting dose of 6-thioguanine in maintenance (60 mg/m$^2$, reduced to 50 mg/m$^2$ after noting 6-thioguanine-related hepatotoxicity). The above mentioned British trial used a starting dose of 40 mg/m$^2$, the COALL-92 trial initially used 50 mg/m$^2$ which was also reduced to 40 mg/m$^2$ upon recognition of marked thrombocytopenia. Although the latter two trials used comparable starting doses, differences with regard to risk of CNS relapse and toxicity can be observed. Thus, the benefit of 6-thioguanine comes with an unacceptable increase in side effects, especially hepatic toxicity.

It is interesting to note that a combination of 6-mercaptopurine and 6-thioguanine has never been envisaged in the art. In particular, it is believed that using only one of the various types of thiopurines is sufficient for treatment since they should act in the same way and, thus, 6-mercaptopurine is regarded as a substitute for 6-thioguanine and vice versa only. The half lives of 6-mercaptopurine and 6-thioguanine are approximately 21 minutes and 90 minutes, respectively, and their plasma areas under the curve are highly variable and not strongly predictive of therapeutic response or side effects. Typical side effects of administering 6-mercaptopurine include diarrhoea, nausea, vomiting, loss of appetite, stomach/abdominal pain, weakness, skin rash, darkening of the skin, or hair loss. Serious adverse reactions include mucositis, fever, sore throat, easy bruising or bleeding, pinpoint red spots on the skin, yellowing of eyes or skin, dark urine, painful or difficult urination. Further, the immune system is depressed, thus, infections may occur more often and more serious. In addition, hepatotoxicity, nephrotoxicity, etc. may occur. Thus, the dosage of thiopurines administered to individuals undergoing cytostatic or chemotherapeutic regimens are restricted. As mentioned before, the enzyme TPMT which is ubiquitously expressed in the cytosol and catalyzes the S-methylation of aromatic and heterocyclic sulfhydryl compounds is an enzyme influencing the balance of 6-thioguanineNs and other metabolites in the individual treated with 6-mercaptopurine or 6-thioguanine. The TPMT locus is subject to genetic polymorphism with heterozygous individuals (6 to 11 % of white individuals) having intermediate TPMT activity and homozygous mutant individuals (0.2 % to 0.6 % of white individuals) having very low TPMT activity. Today about 24 variant alleles have been identified and an overview thereof is given in table 1. Said 24 variant alleles are associated with decreased activity compared with the TPMT wildtype allele. In several independent studies, TPMT genotype showed excellent concordance with TPMT phenotype.

Further, the present inventors reported in Jama, 2005, 293, 1485-1498, about TPMT genotype and early treatment response to 6-mercaptopurine in childhood ALL whereby TPMT genotype has a substantial impact on minimal residual disease after administration of 6-mercaptopurine in the early treatment course of childhood ALL.

TPMT genotype can be used as a surrogate marker of TPMT activity and dose adjustments at least in TPMT deficient patients are required with initial dose reduction to 10 to 15 % of the standard dose of 6-mercaptopurine as described for ALL patients and patients with Crohn's Disease.

Recently, Hardword et al., Cancer Res. 2007, 67, 4965-4972, described that 6-mercaptopurine is more affected by the TPMT polymorphism than 6-thioguanine using a mouse model wherein the TPMT gene was modified by genetic engineering.

However, as mentioned above, substituting 6-mercaptopurine with 6-thioguanine comes with unacceptable hepatic toxicity and, therefore, there is still a need for new therapeuticals having increased efficacy in an individual in need thereof with less side effects.

The present inventors recognized a significantly better event-free survival in TPMT heterozygous patients with childhood ALL compared to TPMT wildtype patients. At the same time, toxicity under exposure towards 6-mercaptopurine did not differ between TPMT heterozygous and TPMT wildtype patients. However, during a two-week late intensification element with the only application of 6-thioguanine at a dose of 60 mg/m$^2$/d, TPMT heterozygous patients had a significantly increased risk of hepatotoxic side-effects. In this analysis, the investigators observed three- to four-fold higher levels of TGNs in TPMT heterozygous patients in comparison to TPMT wildtype patients while methylated thiopurine metabolites were three- to four-fold lower in TPMT heterozygous patients in comparison to TPMT wildtype patients. Considering that a pure increase in 6-mercaptopurine dosage for TPMT wildtype patients will not lead to ratios of TGNs to methylated thiopurine metabolites resembling the treatment situation of TPMT heterozygous individuals in order to equalize treatment outcome in these two subgroups characterized by their different TPMT genotypes, but lead to a preferential increase in methylated metabolites in TPMT wildtype patients, it becomes clear that there is still a need for new therapeuticals having increased efficacy in an individual in need thereof with less side effects. Also, simply switching to 6-thioguanine is not an alternative, as this treatment causes unacceptable hepatotoxic side-effects.

It is therefore an object of the present invention to provide a cytostatic composition having high cytostatic potency and a reduced propensity for causing undesirable side effects by acute and chronic administration thereof.

It is also an object of the invention to provide a method of treatment of individuals in need thereof afflicted with cancer, autoimmune disease or inflammatory bowel disease in which some of the undesirable effects of acute and chronic administration of cytostatics are substantially attenuated.

It is also an object of the invention to provide a kit for determining the strategy of treating cancer, autoimmune disease or inflammatory bowel disease, respectively.

**Summary of the invention**

According to one aspect of the invention, there is provided a cytostatic composition comprising a sub-cytostatic dosage of a first purine analog or a pharmaceutically acceptable salt thereof and a sub-cytostatic dosage of a second purine analog which is different to the first purine analog or a pharmaceutically acceptable salt thereof.

Preferably, the cytostatic composition comprises a sub-cytostatic dosage of 6-mercaptopurine or a pharmaceutically acceptable salt thereof and a sub-cytostatic dosage of 6-thioguanine or a pharmaceutically acceptable salt thereof.

For the purpose of this invention, the term "pharmaceutically acceptable salt" as used herein, refers to salts which are toxicologically safe for human and animal administration. These salts may be selected from a group including hydrochlorides, hydrobromides, hydroiodides, sulfates, bisulfates, nitrates, citrates, tatrates, bitatrates, phosphates, malates, maleates, napsylates, fumarates, succinates, acetates, terephthalates, pamoates and pectinates.

The term "sub-cytostatic dosage" as used herein refers to a dosage of a cytostatic agent which doses does not result in the production of cytostasis when administered to a human or animal alone. The term may be used interchangeably with the term significantly reduced dosage referring to a dosage of a cytostatic agent (e.g., 6-mercaptopurine) which in combination with a second cytostatic agent (e.g., 6-thioguanine) in TPMT wildtype individuals resembles the advantagous treatment effect of individuals heterozygous for a TPMT variant allele conferring reduced TPMT enzyme activity when exposed to 6-mercaptopurine. Sub-cytostatic or significantly reduced dosage refers to a dosage which is at least 50 % below of the recommended dosage of the cytostatic agent when used alone. For example, in case of 6-mercaptopurine the recommended dosage is 50 mg to 200 mg/m$^2$ body surface daily, a sub-cytostatic dosage is, thus, 50 % of the above values, namely, 25 to 100 mg/m$^2$ body surface daily or less. For 6-thioguanine the recommended dosage in treating leukemia is 50 to 200 mg/m$^2$ body surface per day for adults and for children the same dosage or 40 to 100 mg/m$^2$ body surface per day during the induction part of the therapy and in the maintenance phase 40 to 200 mg/m$^2$ body surface per day. The sub-cytostatic dosage is 50 % of the above values.

This term will cover direct administration of the cytostatic compound as well as administration which includes controlled release of the cytostatic compound. Of course, it will be appreciated that a sub-cytostatic dosage of the purine analogs in accordance with the invention will be dependent upon the mode or route of administration thereof.

Suitable cytostatic dosages of such purine analogs may be readily determined by those of skilled in the art. For example, in the case wherein the cytostatic composition comprises 6-mercaptopurine as the first purine analog and 6-thioguanine as the second purine analog or pharmaceutically acceptable salts of said purine analogs, an initial sub-cytostatic dosage of 6-mercaptopurine for an individual human adult through an oral route may be between about 25 and 100 mg/m$^2$ body surface per day depending on the individual to be treated. For 6-thioguanine, the initial sub-cytostatic dosage for an individual human adult through the oral route may be between about 20 and 200 mg/m$^2$ body surface per day. When treating children, the initial sub-cytostatic dosage may be in the range of 25 to 100 mg/m$^2$ body surface for

6-mercaptopurine and 20 to 100 mg/m$^2$ body surface for 6-thioguanine depending on the individual to be treated.

Preferably, the sub-cytostatic dosage is 50 % or less, preferably 30 % or less, e.g. 20 % or less or 10 % or less of the recommended dosage of the cytostatic agent when administered as the sole purine analog. For example, in the case wherein the cytostatic composition comprises 6-mercaptopurine as the first purine analog and 6-thioguanine as the second purine analog or pharmaceutically acceptable salts of said purine analogs, a dosage of 10 % of the recommended dosage of 6-mercaptopurine for human adults through an oral route may be between about 5 and 20 mg/m$^2$ body surface per day. For 6-thioguanine, a dosage of 10 % of the recommended dosage of 6-thioguanine for human adults through an oral route may be between about 5 and 20 mg/m$^2$ body surface per day. When treating children, a dosage of 10 % of the recommended dosage may be in the range of 5 to 20 mg/m$^2$ body surface area for 6-mercaptopurine and 4 to 10 mg/m$^2$ body surface area for 6-thioguanine.

In another aspect, the present invention relates to the use of a sub-cytostatic dosage of a first purine analog or a pharmaceutically acceptable salt thereof in combination with the sub-cytostatic dosage of a second purine analog which is different to the first purine analog or a pharmaceutically acceptable salt thereof for the manufacture of a cytostatic pharmaceutical preparation for simultaneous, separate or sequential administration.

Preferably, the administration is performed concurrently. In this connection, the term "administration concurrently" refers to the administration of a single composition containing both purine analogs or pharmaceutically acceptable salts thereof, or the administration of each such purine analogs as separate compositions and/or delivered by separate routes within a short enough period of time that the effective result is equivalent to that obtained when both purine analogs are administered as a single composition.

In another aspect, the present invention relates to methods of treatment comprising in a first step phenotyping the individual afflicted with cancer, autoimmune disease, or inflammatory bowel disease, in particular, with leukemia for the presence or absence of a TPMT polymorphism and, in a second step, determining the effective amount of the first purine analog and the second purine analog or suitable salts thereof to be administered to said individual in need thereof based on the TPMT genotype or phenotype. Said effective amount of the first purine analog and the second purine analog are administered simultaneously, separately or sequentially, preferably concurrently to the individual in need thereof.

Finally, the present invention relates to kits comprising the cytostatic composition according to the present invention together with means for determining a polymorphism in the enzyme TPMT.

**Brief description of the drawings**

Figure 1: scheme of the metabolic pathway of 6-mercaptopurine (MP) and 6-thioguanine (TG)

Figure 2. Mean viable cells of a TPMT wild-type lymphoblastoid cell line after 72 h exposure towards different concentrations of 6-mercaptopurine (MP) and 6-thioguanine (TG) under standard cell culture conditions. The exact viability rates for assessment of the observed slight supraadditive effect are given in table 2.

**Detailed description of the present invention**

The present invention relates to a cytostatic composition comprising a cytostatic dosage of a first purine analog or a pharmaceutically acceptable salt thereof and a sub-cytostatic dosage of a second purine analog which is different to the first purine analog or a pharmaceutically acceptable salt thereof. Said cytostatic compositions are especially useful for treating cancer, autoimmune diseases, or inflammatory bowel diseases. In a preferred embodiment said cytostatic compositions according to the present invention are used to treat leukemia.

The term "leukemia" according to the invention comprises a cancer of blood-forming tissues of the bone marrow. It is characterized by the growth of immature white blood cells. The disease can be categorized based on which blood cells are abnormal. Lymphomas are cancers of lymphoid tissue (lymphnodes, spleen and other organs or the immune system). In a particular preferred embodiment, the cytostatic composition is useful for treating acute lymphoblastic leukemia and in another embodiment acute myelogenous leukemia, preferably in children.

The acute lymphoblastic leukemia is the most frequent type of cancer in childhood amounting to 25 % of all cases.

As used herein, the term "subject" or "individual" or "patient" which is used herein interchangeably, refers to an individual or a subject or a patient in need of a therapy or suspected to be afflicted with a condition or disease, like cancer, autoimmune disease or inflammatory bowel disease, for example leukemia, rheumatoid arthritis or Crohn's Disease. Preferably, the subject or individual is a vertebrate and more preferred a mammal, particular preferred a human.

In a preferred embodiment, the first purine analog is 6-mercaptopurine or a pharmaceutically acceptable salt thereof or its prodrug, namely azathioprine.

In another preferred embodiment, the second purine analog is 6-thioguanine or a pharmaceutically acceptable salt thereof.

Preferably, the initial sub-cytostatic dosage of the purine analog in case of 6-mercaptopurine as a first purine analog for a human individual is 50 %, like 30 %, 20 % or preferably 10 % of the recommended dosage administered when using 6-mercaptopurine as a purine analog alone.

Similarly, when using 6-thioguanine as a purine analog, the initial sub-cytostatic dosage of 6-thioguanine or any other purine analog for a human individual is 50 %, like 30 %, 20 % or preferably 10 % of the recommended dosage administered when using 6-thioguanine as purine analog alone.

In a preferred aspect, the cytostatic composition according to the present invention contains an initial sub-cytostatic dosage of 6-mercaptopurine for a human individual through the oral route of between 10 mg and 100 mg/m$^2$/day and an initial sub-cytostatic dosage of 6-thioguanine for a human individual through the oral route of between 5 mg and 50 mg/m$^2$/day.

The cytostatic composition may be in a form adapted for simultaneous, separate or sequential administration of the first purine analog or a pharmaceutically acceptable salt thereof and the second purine analog or a pharmaceutically acceptable salt thereof. Preferably, the two components of the cytostatic composition are administered concurrently.

In another preferred embodiment, the cytostatic composition is in a slow- or controlled-release dosage form.

In another aspect, the present invention relates to the use of a sub-cytostatic dosage of a first purine analog or a pharmaceutically acceptable salt thereof in combination with a sub-cytostatic dosage of a second purine analog which is different to the first purine analog or a pharmaceutically acceptable salt thereof for the manufacture of a cytostatic pharmaceutical preparation for simultaneous, separate or sequential administration.

In a preferred embodiment, 6-mercaptopurine or a pharmaceutically acceptable salt and/or 6-thioguanine or a pharmaceutically acceptable salt thereof are used for the preparation of said cytostatic pharmaceutical preparation. Preferably, the sub-cytostatic dosage of the purine analog, like 6-mercaptopurine, its prodrug, azathioprine or 6-thioguanine is 50 % or less, like 30 %, 20 % or 10 % or less of the recommended dosage administered when using said purine analog alone.

In another embodiment, the sub-cytostatic dosage is depending on the route of administration and when including intravenous application may range between 10 and 1000 mg/m$^2$/d for 6-mercaptopurine, 5 mg and 600 mg/m$^2$/d for 6-thioguanine and 10 and 1600 mg/m$^2$/d for azathioprine.

The cytostatic composition can be administered on various ways, e.g. oral, rectal, parenteral, sub-lingual, bucal, intradecal, epithoral, intravenous, intra-articular, intramuscular, intra-dermal, subcutaneous, inhalational, intra-ocular, intra-peritonal, intra-cerebroventricular, transdermal. Preferably, the composition is administered orally, sublingually, injected intravenously, subcutaneously, into the muscle, fat or other tissues, administered topically onto the skin or onto a mucosa, inhaled as aerosol into the lungs or administered rectal.

Particular preferred are administration via the oral or intravenous route.

The cytostatic composition according to the present invention can be provided in suitable dosage forms. Dosage forms include tablets, pills, capsules, powders, granulates, salves, plasters releasing substances transdermal to the individual, tinctures, uvula, suspensions, solutions, injections, dispersions, syrups, troches, aerosols and the like. These dosage forms may also include injecting or implanting slow releasing devices designed specifically for the purpose or other forms of implants modified to act additionally in this fashion. Slow- or controlled-release of the cytostatic agents may be effected by coating the same, for example with hydrophobic polymers including acrylic resins, waxes, higher aliphatic alcohols, polytactic and polyglycolic acids and cellulose derivative, such as hydropropylmethylcellulose. In addition, the controlled release may be effected by using a polymer matrizes, liposomes and/microspheres.

The cytostatic composition can be applied to an individual one or more times daily, weekly, monthly, or longer time intervals. The composition according to the present invention can be used alone or in combination with other pharmaceutical composition typically used in regimens fighting cancer, autoimmune disease or inflammatory bowel disease, in particular, leukemia. In particular, the cytostatic compositions according to the present invention can be used in combination with known therapeutica for the treatment of leukemia and cytostatic agent and irradiation.

Thus, the pharmaceutical composition may be administered with a physiological acceptable carrier to a patient. In a specific embodiment, the term "pharmaceutically acceptable" means approved by regulatory agency or other generally recognized pharmacopayer for use in animals, and more particular, in humans. The term "carrier" refers to a diluent, adjuvant, excipient or vehicle with which the therapeutic is administered.

Typically, pharmaceutically or therapeutically acceptable carrier is a carrier medium which does not interfere with the effectiveness of the biological activity of the active ingredients and which is not toxic to the host or patient. Depending upon the particular route of administration, a variety of pharmaceutically acceptable carriers well known in the art may be used. These carriers may be selected from a group including sugars, starches, cellulose and its derivatives, malt, gelatine, talc, calciumsulphate, vegetable oils, synthetic oils, polyols, algenic acid, phosphate buffer, solutions, emulsifiers, isotonic saline and pyrogene free water.

Pharmaceutical compositions of the present invention suitable for oral or parenteral administration may be presented as solid units such as capsules, such as tablets each containing a predetermined amount of each of the purine analog as a powder or granules or as a solution or a suspension in aqueous liquid, a non-aqueous liquid, an oil water emulsion or a water in oil liquid emulsion. Such compositions may be prepared by any of the methods of pharmacy but all methods include the step of bringing into association the sub-cytostatic dosages of each of the purine analogs as described above with the carrier which constitutes one or more necessary ingredients. In general, the compositions are prepared by uniform and intimately admixing the cytostatic agent with liquid carriers or finely dividend solid carriers or both, and then, if necessary, shaping the product into the desired presentation.

In the context of the present invention the term "therapeutically effective amount" is a dose that produces the effects for which it is administered. The exact dose will depend on the focus of the treatment, and will be ascertainable by one skilled in the art using known techniques. As it is known in the art and described above, adjustment of systemic versus localized delivery, age, body weight, general health, sex, diet, time of administration, drug interaction and the variety of the condition may be necessary and will be ascertainable with routine experimentation by those skilled in the art.

The cytostatic composition according to the present invention is particularly useful for treating leukemia, in particular, childhood leukemia, like acute lymphoblastic leukemia or acute myeloid leukemia. In another aspect, the compounds of the present invention are used for the treatment of autoimmune diseases, like rheumatoid arthritis. Further, the cytostatic compositions according to the present invention are useful for treating inflammatory bowel disease, like Crohn's disease or ulcerative colitis.

In still another embodiment, the present invention relates to methods of treating subjects afflicted with cancer like malignant neoplasias, in particular, leukemia, autoimmune disease and inflammatory bowel disease. In particular, the present invention relates to methods for treating neoplasias like leukemia, especial like childhood leukemia like acute lymphoblastic leukemia or acute myeloid leukemia.

Said treatment comprises the steps of determining the TPMT genotype or phenotype of said individual afflicted with said disease. Based on the TPMT genotype or phenotype, the effective amount of the first purine analog and the second purine analog to be administered to said individual in need thereof is determined. Said effective amounts are administered to said individual, said administration of these two purine analogs may be effected simultaneously, separately, or sequentially, for example concurrently.

In particular, the TPMT genotype is determined. The genotype of the TPMT enzyme may be homozygous for both wildtype alleles, heterozygous, namely having one wildtype allele and one allele containing a genetic variant, and homozygous for a variant of the TPMT gene. It is known that genetic variation at the TPMT locus influences the metabolism of 6-mercaptopurine since TPMT is involved in the biochemical pathway of 6-mercaptopurine. Depending on the TPMT genotype it has been found that heterozygous and wildtype patients having acute lymphoblastic leukemia demonstrate a different rate of leukemic cell reduction after administration of 6-mercaptopurine. In comparison to TPMT wildtype patients, heterozygous individuals show a faster reduction of tumor cells under 6-mercaptopurine therapy and, finally, demonstrate a significantly higher long-term event-free survival rate.

TPMT activity is inadvertently related to the concentration of active thioguanine metabolites after administration of thiopurines. The rare homozygous deficient individuals develop extreme myelosuppression which can be fatal when given usual doses of thiopurines, necessitating a ten fold dosage decrease to prevent such toxicity. In contrast, heterozygotes, constituting about 10 % of the white population, often exhibit an intermediate degree of toxicity following 6-mercaptopurine. Homozygous wildtype patients having a high activity of TPMT may be at increased risk of poor treatment response due to fast degradation of 6-mercaptopurine. However, increasing the doses of 6-mercaptopurine in TPMT wildtype patients is not possible due to toxicity and cancer of 6-mercaptopurine.

The inventors found that a combination of 6-mercaptopurine and 6-thioguanine enables to treat patients wildtype for TPMT without the necessity to increase the 6-mercaptopurine doses. It was found that 6-mercaptopurine and 6-thioguanine act independently and combining these two purine analogs increases the efficacy of a thiopurine based therapy in particular in TPMT wildtype patients. It has been identified that when administering identical doses of 6-mercaptopurine in TPMT heterozygous patients, larger amounts of the thioguanine nucleotides are available and, thus, the rate of remission of the tumor is higher due to a lower rate of degradation of 6-mercaptopurine via the TPMT enzyme to methylated metabolites. However, at the same time, the level of methylated metabolites is lower in heterozygous patients as well as in patients being homozygous for a genetic variant of TPMT. Methylated metabolites of 6-mercaptopurine have also a therapeutic activity based on the inhibition of the de novo synthesis of purines. To have an optimal therapy to patients having different TPMT forms the therapeutic regimen must be adapted to the individual TPMT genotype of the subject in need thereof. That is, for example in TPMT wildtype patients the combination of different types of thiopurines, namely, the administration of 6-mercaptopurine in combination with 6-thioguanine enables to obtain a desired ratio of methylated metabolites derived mainly from the 6-mercaptopurine and cytotoxic thioguanine nucleotides stemming from the 6-mercaptopurine as well as the 6-thioguanine, thus, receiving an improved efficacy in comparison to a monotherapy with 6-mercaptopurine or 6-thioguanine.

In particular, the inventors found that when combining two different purine analogs, e.g. 6-mercaptopurine or its prodrug azathioprine and 6-thioguanine it is possible to reduce the dosage to be administered to a patient, thus, decreasing the risk of adverse side effects while increasing the therapeutic efficiency. As shown below in the examples, a combination of the two different purine analogs allows to significantly reduce the dosage of the single components of said compounds. Contrary to the general opinion in the art that the purine analogs 6-mercaptopurine and 6-thioguanine have the same effect in therapy and, thus, a combination thereof would not result in better therapy, the inventors found that additives and synergistics effects can be observed when combining these two purine analogs. That is, a therapy based on the combination of the two purine analogs display the advantage of a synergistic amplification of the activity of the single substances, thus, allowing to reduce the toxicity while using sub-cytostatic dosages of said purine analogs while achieving identical or better therapeutic efficiency.

In particular, in TPMT wildtype patients the combination of the two purine analogs is extremely useful when treating cancer, autoimmune diseases or inflammatory bowel diseases. The TPMT polymorphism (table 1) may be determined by means known in the art.

**Table 3.** Phenotypically relevant variant *TPMT* alleles

| Alleles | Exon | Mutation | Amino acid change | Reference |
|---|---|---|---|---|
| *1S | 7 | 474T>C | - | 1a |
| *2 | 5 | 238G>C | Ala[80]Pro | 2 |
| *3A | 7<br>10 | 460G>A<br>719A>G | Ala[154]Thr<br>Tyr[240]Cys | 3/4 |
| *3B | 7 | 460G>A | Ala[154]Thr | 3/4 |
| *3C | 10 | 719A>G | Tyr[240]Cys | 3/4 |
| *3D | 5<br>7<br>10 | 292G>T<br>460G>A<br>719A>G | Glu[98]X<br>Ala[154]Thr<br>Tyr[240]Cys | 5 |
| *4 | - | G>A transition that disrupts the intron/exon acceptor splice junction at the final 3' nucleotide of intron 9 | | 5 |
| *5 | 4 | 146T>C | Leu[49]Ser | 5 |
| *6 | 8 | 538A>T | Tyr[180]Phe | 5 |
| *7 | 10 | 681T>G | His[227]Gln | 6 |
| *8 | 10 | 644G>A | Arg[215]His | 7 |
| *9 | 5 | 356A>C | Lys[119]Thr | 8 |
| *10 | 7 | 430G>C | Gly[144]Arg | 9 |
| *11 | 6 | 395G>A | Cys[132]Tyr | 10 |
| *12 | 6 | 374C>T | Ser[125]Leu | 11 |
| *13 | 3 | 83A>T | Glu[28]Val | 11 |
| *14 | 3 | 1A>G | Met[1]Val | 12 |
| *15 | - | G>A transition that disrupts the intron/exon acceptor splice junction at the final 3' nucleotide of intron 7 | | 12 |
| *16 | 7 | 488G>A | Arg[163]His | 8 |
| *17 | 3 | 124C>G | Gln[42]Glu | 8 |
| *18 | 4 | 211 G>A | Gly[71]Arg | 8 |
| *19 | 5 | 365A>C | Lys[122]Glu | 13 |
| *20 | 10 | 712A>G | Lys[238]Glu | 14 |
| *21 | 4 | 205C>G | Leu[69]Val | 14 |

(continued)

| Alleles | Exon | Mutation | Amino acid change | Reference |
|---------|------|----------|-------------------|-----------|
| *22 | 7 | 488G>C | Arg[163]Pro | 14 |

a frequently observed silent change, reported allele frequency 0.215

References:

1. Alves S, Prata MJ, Ferreira F, Amorim A., Pharmacogenetics 1999;9:257-261
2. Tai HL, Krynetski EY, Yates CR, et al., Am J Hum Genet 1996;58:694-702.
3. Szumlanski S, Otterness D, Her C, et al. , DNA Cell Biol 1996;15:17-30.
4. Tai HL, Krynetski EY, Yates CR, et al., Am J Hum Genet 1996;58:694-702.
5. Otterness D, Szumlanski C, Lennard L, et al., Clin Pharmacol Ther 1997;62:60-73.
6. Spire-Vayron de la Moureyre C, Debuysere H, Sabbagh N, et al., Hum Mutat 1998; 12:177-185.
7. Hon YY, Fessing MY, Pui CH, Relling MV, Krynetski EY, Evans WE., Hum Mol Genet 1999;8:371-376.
8. Schaeffeler E, Fischer C, Brockmeier D, et al., Pharmacogenetics 2004;14:407-417.
9. Colombel JF, Ferrari N, Debuysere H, et al., Gastroenterology 2000;118:1025-1030.
10. Schaeffeler E, Stanulla M, Greil J, Schrappe M, Eichelbaum M, Zanger UM, Schwab M., Leukemia 2003;17: 1422-1424.
11. Hamdan-Khalil R, Allorge D, Lo-Guidice JM, et al., Biochem Biophys Res Commun 2003;309:1005-1010.
12. Lindqvist M, Haglund S, Almer S, et al., Pharmacogenetics 2004;14:261-265.
13. Hamdan-Khalil R, Gala JL, Allorge D, et al., TPMT*16 and TPMT*19. Biochem Pharmacol 2005;69:525-529.
14. Schaeffeler E, Eichelbaum M, Reinisch W, Zanger UM, Schwab M., Hum Mutat 2006;27:976.

For example, the polymorphism may be determined by different assays including restriction fragment length polymorphism analysis after polymerase chain reaction (RFLP-PCR), denaturing high-performance liquid chromatography (HPLC) methods, real-time PCR analysis, molecular haplotyping, a multiplex amplification refractory mutation system (ARMS) strategy, arrayed primer extension (APEX), pyrosequencing, DNA microarray, and other PCR-based techniques and/or mass-spectroscopy-based techniques. TPMT activity can also be analyzed directly by different phenotyping assays including the radiochemical method developed by Weinshilboum and colleagues and more recent non-radioactive HPLC methods using either 6-mercaptopurine or 6-thioguanine as substrates.

Thus, the kit according to the present invention comprises means for determining the presence or absence of a TPMT genetic variant beside the cytostatic composition according to the present invention. That is, when stratifying a subject to determine the therapy regimen for the treatment of cancer, autoimmune disease or inflammatory bowel disease, in particular of malignant neoplasias like leukemia, the stratification includes determining the TPMT genotype or phenotype and, subsequently, determining the dosage of the first purine analog, like 6-mercaptopurine or azathioprine and the dosage of the second purine analog, like 6-thioguanine.

The purine analogs useful according to the present invention, in particular, 6-mercaptopurine or its prodrugs azathioprine and 6-thioguanine are commercially available as single substances. It is possible to administer said purine analogs in its commercial (packing) forms or to produce solid or liquid preparations containing the purine analogs as active ingredients. In case of a solution for injection, it is possible to provide injectable forms containing already the combination of both purine analogs or to provide composites wherein two separate units containing one of the purine analogs are provided, thus, separate and sequential administration of the purine analogs.

Preferably, the molar ratio of 6-mercaptopurine to 6-thioguanine in a cytostatic preparation according to the present invention is 1:200 to 200:1.

Depending on the TPMT genotype the cytostatic composition according to the present invention contains higher amounts of 6-thioguanine, in case of TPMT wildtype patients and lower amounts of 6-thioguanine in case of patients carrying TPMT genetic variants.

By pre-screening of the TPMT genotype or phenotype of the patients, it is possible to stratify the patient afflicted with a disease for the optimal therapeutic regimen treating the same.

**Example**

The following examples are provided to illustrate the invention further. However, they are not meant to delimit the scope of the invention in any way.

Human CCRF-CEM cells (T-cell human acute lymphoblastic leukemia) were purchased from the American Type Culture Collection (Rockville, MD). Lymphoblastoid cell lines (523, 598, 607, 718) were established from normal donors and obtained from the Institute of Transfusion Medicine, Hannover Medical School, Germany. Cells were grown in RPMI 1640 (PAA, Pasching, Austria) supplemented with 10% heat-inactivated fetal bovine serum and 2 mM glutamine. All cells were maintained in log phase at 37°C in a 5% CO2 humidified atmosphere.

6-mercaptopurine and 6-thioguanine were purchased from Sigma-Aldrich (St. Louis, MO). 6-mercaptopurine was dissolved in RPMI 1640 and filtered (0.45 $\mu$m filter); the concentration was calculated using the UV molar extinction coefficient (340 = 19600); 6-thioguanine, was dissolved in DMSO (final concentration always less than 0.1% in the culture media). Cells treated with drugs were compared with cells treated with solvent alone. Cell viability was assessed using the trypan blue exclusion test. This dye exclusion test is used to determine the number of viable cells present in a cell suspension and is based on the principle that live cells possess intact cell membranes that exclude certain dyes, such as trypan blue, whereas dead cells do not. In this test, a cell suspension is simply mixed with dye and then visually examined to determine whether cells take up or exclude dye. A viable cell will have a clear cytoplasm whereas a nonviable cell will have a blue cytoplasm.

Cytotoxicity was expressed as the percentage of viable cells and calculated as follows:

$$\text{Viable cells (\%)} = \frac{\text{the total number of viable cells per ml of aliquot}}{\text{The total number of cells per ml of aliquot}} \times 100$$

Results were expressed as mean $\pm$ SD. Evaluated data represented the average of at least three independent experiments. Data are shown in table 2 below.

**Table 2.** Mean survival of TPMT wildtype lymphoblastoid cells exposed towards 6-mercaptopurine (MP) or 6-thioguanine (TG) alone or a combination of both.

| Dose MP ($\mu$M) | Dose TG ($\mu$M) | n | Mean percentage viable cells after 72 h | Standard deviation |
|---|---|---|---|---|
| 1 | - | 9 | 73.1 | 1.90 |
| 0.5 | - | 9 | 79.8 | 1.48 |
| 0.25 | - | 9 | 84.7 | 1.00 |
| 0.125 | - | 9 | 89.3 | 2.50 |
| | | | | |
| - | 0.25 | 9 | 71.3 | 1.41 |
| - | 0.125 | 9 | 75.9 | 1.05 |
| - | 0.0625 | 9 | 81.2 | 1.20 |
| | | | | |
| 1 | 0.25 | 9 | 42.3 | 2.35 |
| 1 | 0.125 | 9 | 44.8 | 1.92 |
| 1 | 0.0625 | 9 | 49.6 | 1.88 |
| | | | | |
| 0.5 | 0.25 | 9 | 45.9 | 1.27 |
| 0.5 | 0.125 | 9 | 51.3 | 1.00 |
| 0.5 | 0.0625 | 9 | 56.0 | 1.00 |
| | | | | |
| 0.25 | 0.25 | 9 | 52.1 | 1.97 |
| 0.25 | 0.125 | 9 | 54.8 | 1.30 |

(continued)

| Dose MP ($\mu$M) | Dose TG ($\mu$M) | n | Mean percentage viable cells after 72 h | Standard deviation |
|---|---|---|---|---|
| 0.25 | 0.0625 | 9 | 59.0 | 1.50 |
| | | | | |
| 0.125 | 0.25 | 9 | 55.2 | 0.97 |
| 0.125 | 0.125 | 9 | 60.6 | 1.13 |
| 0.125 | 0.0625 | 9 | 64.3 | 1.73 |

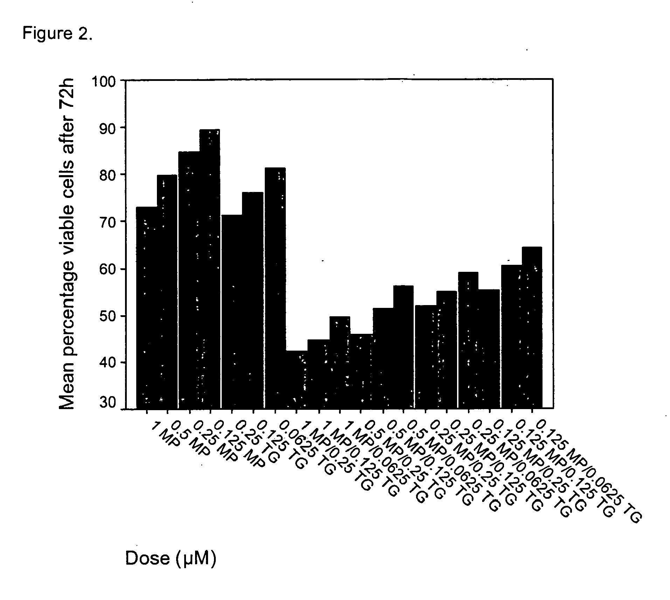

As shown in figure 2, a combination of TG and MP allows reducing significantly the required amount of each of the components to reduce the number of viable cells.

## Claims

1. A cytostatic composition comprising a sub-cytostatic dosage of a first purine analog or a pharmaceutically acceptable salt thereof and a sub-cytostatic dosage of a second purine analog which is different to the first purine analog or a pharmaceutically acceptable salt thereof.

2. The cytostatic composition according to claim 1, wherein the first purine analog is 6-mercaptopurine or azathioprine or a pharmaceutically acceptable salt thereof.

3. The cytostatic composition according to claim 1 or 2, wherein the second purine analog is 6-thioguanine or a pharmaceutically acceptable salt thereof.

4. A cytostatic composition as claimed in claim 1, wherein the initial sub-cytostatic dosage of the purine analog for a human individual is 50 percent or less of the recommended dosage to be administered when using said purine analog alone.

5. A cytostatic composition according to claim 2, wherein the initial sub-cytostatic dosage of 6-mercaptopurine for a human individual through the oral route is between 25 mg and 100 mg/m$^2$ per day or less.

6. A cytostatic composition according to claim 3, wherein the initial sub-cytostatic dosage of 6-thioguanine for a human individual through the oral route is between 20 mg and 100 mg/m$^2$ per day or less.

7. A cytostatic composition according to any one of the preceding claims adapted for simultaneous, separate or sequential administration of the first purine analog or a pharmaceutically acceptable salt thereof and the second purine analog or a pharmaceutically acceptable salt thereof.

8. A cytostatic composition as claimed in any one of the preceding claims, wherein said composition is in a slow- or controlled-released dosage form.

9. The use of a sub-cytostatic dosage of a first purine analog or a pharmaceutically acceptable salt thereof in combination with a sub-cytostatic dosage of a second purine analog which is different to the first purine analog or a pharmaceutically acceptable salt thereof for the manufacture of a cytostatic pharmaceutical preparation for simultaneous, separate or sequential administration.

10. An use according to claim 9, wherein the first purine analog is 6-mercaptopurine or a pharmaceutically acceptable salt thereof.

11. An use according to claim 9, wherein the second purine analog is 6-thioguanine or a pharmaceutically acceptable salt thereof.

**12.** An use of according to claim 9, wherein the initial sub-cytostatic dosage of the purine analog for a human individual *is 50 percent* or less of the recommended dosage administered when using said purine analog alone.

**13.** An use according to claim 9 or 10, wherein the initial sub-cytostatic dosage of 6-mercaptopurine for a human individual through the oral route is between 25 and 100 mg or less/m$^2$ body surface per day.

**14.** An use according to claim 9 or 10, wherein the initial sub-cytostatic dosage for 6-thioguanine for a human adult through the oral route may be between about 20 and 100 mg or less/m$^2$ body surface per day.

**15.** An use according to claim 9, wherein the mode of administering the composition is selected from the group consisting of oral, rectal, parentaral, sub-lingual, bucal, intradecal, epithoral, intravenous, intra-articular, intramuscular, intra-dermal, subcutaneous, inhalational, intra-ocular, intra-peritonal, intra-cerebroventricular, transdermal.

**16.** An use according to any one of claims 9 to 15, for the manufacture of a pharmaceutical preparation for the treatment of cancer, autoimmune disease, or inflammatory bowel disease.

**17.** An use according to claim 16 for the treatment of leukemia.

**18.** An use according to claim 17, wherein the leukemia is childhood leukemia.

**19.** An use according to claim 18, wherein the childhood leukemia is ALL or AML.

**20.** An use according to claim 16 for the treatment of psoriasis or psoriatic arthiritis, or rheumatiod arthritis.

**21.** An use according to claim 16 for the treatment of Crohn's disease or ulcerative colitis.

**22.** Kit comprising a composition according to any one of claims 1 to 8 and means for determining a polymorphism in the enzyme thiopurine S-methyltransferase (TPMT).

**23.** Kit according to claim 22, wherein the TPMT polymorphism is any one of the polymorphisms shown below:

| Alleles | Exon | Mutation | Amino acid change |
|---------|------|----------|-------------------|
| *1S | 7 | 474T>C | - |
| *2 | 5 | 238G>C | Ala$^{80}$Pro |
| *3A | 7<br>10 | 460G>A<br>719A>G | Ala$^{154}$Thr<br>Tyr$^{240}$Cys |
| *3B | 7 | 460G>A | Ala$^{154}$Thr |
| *3C | 10 | 719A>G | Tyr$^{240}$Cys |
| *3D | 5<br>7<br>10 | 292G>T<br>460G>A<br>719A>G | Glu$^{98}$X<br>Ala$^{154}$Thr<br>Tyr$^{40}$Cys |
| *4 | - | G>A transition that disrupts the intron/exon acceptor splice junction at the final 3' nucleotide of intron 9 | |
| *5 | 4 | 146T>C | Leu$^{49}$Ser |
| *6 | 8 | 538A>T | Tyr$^{180}$Phe |
| *7 | 10 | 681 T>G | His$^{227}$Gln |
| *8 | 10 | 644G>A | Arg$^{215}$His |

(continued)

| Alleles | Exon | Mutation | Amino acid change |
|---------|------|----------|-------------------|
| *9 | 5 | 356A>C | Lys$^{119}$Thr |
| *10 | 7 | 430G>C | Gly$^{144}$Arg |
| *11 | 6 | 395G>A | Cys$^{132}$Tyr |
| *12 | 6 | 374C>T | Ser$^{125}$Leu |
| *13 | 3 | 83A>T | Glu$^{28}$Val |
| *14 | 3 | 1A>G | Met$^{1}$Val |
| *15 | - | G>A transition that disrupts the intron/exon acceptor splice junction at the final 3' nucleotide of intron 7 | |
| *16 | 7 | 488G>A | Arg$^{163}$His |
| *17 | 3 | 124C>G | Gln$^{42}$Glu |
| *18 | 4 | 211 G>A | Gly$^{71}$Arg |
| *19 | 5 | 365A>C | Lys$^{122}$Glu |
| *20 | 10 | 712A>G | Lys$^{238}$Glu |
| *21 | 4 | 205C>G | Leu$^{69}$Val |
| *22 | 7 | 488G>C | Arg$^{163}$Pro |

**24.** A kit according to claims 22 or 23, wherein the means for determining the presence of TPMT genetic variation comprises means for detecting the polymorphisms by PCR.

**25.** The use according to any one of claims 9 to 21, wherein the effective amount of the first purine analog or pharmaceutical salt thereof and of the second purine analog or pharmaceutically acceptable salt thereof depend on the TPMT polymorphism of the individual in need of cytostatic treatment.

**26.** A method for treating an individual afflicted with cancer, autoimmune disease, or inflammatory bowel disease, in particular with leukemia, comprising the step of a) determining the TPMT phenotype or genotype of said individual and b) determining the effective amount of the first purine analog and second purine analog to be administered to said individual in need thereof based on the TPMT genotype or phenotype, c) administering said effective amount of a first purine analog, preferably 6-mercaptopurine and d) administering simultaneously, separately or sequentially said effective amount of a second purine analog which is different to the first purine analog or a pharmaceutically acceptable salt thereof, preferably 6-thioguanine, wherein the dosage of the first and second purine analog is lower than the effective dosage of said purine analogs when administered alone.

**27.** The method according to claim 26, wherein when the TPMT genotype or phenotype is a homozygous wildtype, the individual in need thereof receives higher doses of the first and the second purine analog compared to a person being heterozygous or homozygous for a polymorphism of the TPMT gene.

**28.** The method according to claims 26 or 27 wherein the first purine analog is 6-mercaptopurine or azathioprine and the second purine analog is 6-thioguanine.

Figure 1

Figure 2.

Europäisches
Patentamt

European
Patent Office

Office européen
des brevets

**PARTIAL EUROPEAN SEARCH REPORT**   Application Number

which under Rule 63 of the European Patent Convention EP 08 00 3920
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HENDERSON J F ET AL: "Potentiation of carcinostasis by combinations of thioguanine and 6-mercaptopurine." BIOCHEMICAL PHARMACOLOGY OCT 1960, vol. 5, October 1960 (1960-10), pages 167-168, XP002498709 ISSN: 0006-2952 * the whole document * * table 1 * | 1-16 | INV. A61K31/52 A61P35/02 A61P1/04 A61P29/00 |
| Y | | 1-28 | |
| X | KELA U ET AL: "Potentiation of the inhibition of xanthine oxidase by a combination of 6-mercaptopurine and 6-thioguanine" EXPERIENTIA 1981 CH, vol. 37, no. 2, 1981, pages 175-176, XP009106706 ISSN: 0014-4754 | 1-16 | |
| Y | * the whole document * * tables 1,2 * | 1-28 | TECHNICAL FIELDS SEARCHED (IPC) |
| | -/-- | | A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 8 October 2008 | Veronese, Andrea |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04E07)

Europäisches
Patentamt
European
Patent Office
Office européen
des brevets

**PARTIAL EUROPEAN SEARCH REPORT**

Application Number

EP 08 00 3920

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| Y | EVANS W E ET AL: "Mercaptopurine vs thioguanine for the treatment of acute lymphoblastic leukemia" LEUKEMIA RESEARCH, NEW YORK,NY, US, vol. 18, no. 11, 1 November 1994 (1994-11-01), pages 811-814, XP022922253 ISSN: 0145-2126 [retrieved on 1994-11-01] See page 811, column 1, passage reciting "there may be reasons to consider 6TG as an addition and not a replacement for 6MP" Page 813, column 1, passage mentioning the strategy of using 6MP and 6TG in combination ----- | 1-28 | |
| Y | LENNARD L ET AL: "Individualizing therapy with 6-mercaptopurine and 6-thioguanine related to the thiopurine methyltransferase genetic polymorphism." THERAPEUTIC DRUG MONITORING AUG 1996, vol. 18, no. 4, August 1996 (1996-08), pages 328-334, XP009106713 ISSN: 0163-4356 * abstract * See page 331-332: TPM activity and TGN formation ----- | 1-28 | TECHNICAL FIELDS SEARCHED (IPC) |
| Y | HARTFORD CHRISTINE ET AL: "Differential effects of targeted disruption of thiopurine methyltransferase on mercaptopurine and thioguanine pharmacodynamics." CANCER RESEARCH 15 MAY 2007, vol. 67, no. 10, 15 May 2007 (2007-05-15), pages 4965-4972, XP002498710 ISSN: 0008-5472 * the whole document * ----- -/-- | 1-28 | |

EPO FORM 1503 03.82 (P04C10)

Europäisches
Patentamt

European
Patent Office

Office européen
des brevets

PARTIAL EUROPEAN SEARCH REPORT

Application Number

EP 08 00 3920

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| D,A | STANULLA ET AL: "Thioguanine versus mercaptopurine in childhood ALL" LANCET THE, LANCET LIMITED. LONDON, GB, vol. 368, no. 9544, 14 October 2006 (2006-10-14), pages 1304-1306, XP005706211 ISSN: 0140-6736 * the whole document * ----- | 1-28 | |
| A | HEMLATA TAMTA ET AL: "Nature and position of functional group on thiopurine substrates influence activity of xanthine oxidase - Enzymatic reaction pathways of 6-mercaptopurine and 2-mercaptopurine are different" BIOCHEMISTRY (MOSCOW), KLUWER ACADEMIC PUBLISHERS-PLENUM PUBLISHERS, NE, vol. 72, no. 2, 1 February 2007 (2007-02-01), pages 170-177, XP019478052 ISSN: 1608-3040 * the whole document * ----- | 1-28 | TECHNICAL FIELDS SEARCHED (IPC) |
| A | PETIT ET AL: "Differential toxic effects of azathioprine, 6-mercaptopurine and 6-thioguanine on human hepatocytes" TOXICOLOGY IN VITRO, ELSEVIER SCIENCE, GB, vol. 22, no. 3, 25 January 2008 (2008-01-25), pages 632-642, XP022511899 ISSN: 0887-2333 * the whole document * ----- | 1-28 | |

EPO FORM 1503 03.82 (P04C10)

Europäisches
Patentamt

European
Patent Office

Office européen
des brevets

**INCOMPLETE SEARCH
SHEET C**

Application Number

EP 08 00 3920

Although claims 26-28 are directed to a method of treatment of the
human/animal body (Article 53(c) EPC), the search has been carried out
and based on the alleged effects of the compound/composition.

     -----

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **van Lohn et al.** *Journal of Pharmacology and Experimental Therapeutics,* 1987, vol. 242, 21-26
- **Harms et al.** *Blood,* 2003, vol. 102, 2736-2740
- **Vora.** *Lancet,* 2006, vol. 368, 1339-1348
- **Stork et al.** *Blood,* 2002, vol. 100, 156a
- *Jama,* 2005, vol. 293, 1485-1498
- **Hardword et al.** *Cancer Res.,* 2007, vol. 67, 4965-4972
- **Alves S ; Prata MJ ; Ferreira F ; Amorim A.** *Pharmacogenetics,* 1999, vol. 9, 257-261
- **Tai HL ; Krynetski EY ; Yates CR et al.** *Am J Hum Genet,* 1996, vol. 58, 694-702
- **Szumlanski S ; Otterness D ; Her C et al.** *DNA Cell Biol,* 1996, vol. 15, 17-30
- **Tai HL ; Krynetski EY ; Yates CR et al.** *Am J Hum Genet,* 1991, vol. 58, 694-702
- **Otterness D ; Szumlanski C ; Lennard L et al.** *Clin Pharmacol Ther,* 1997, vol. 62, 60-73
- **Spire-Vayron de la Moureyre C ; Debuysere H ; Sabbagh N et al.** *Hum Mutat,* 1998, vol. 12, 177-185
- **Hon YY ; Fessing MY ; Pui CH ; Relling MV ; Krynetski EY ; Evans WE.** *Hum Mol Genet,* 1999, vol. 8, 371-376
- **Schaeffeler E ; Fischer C ; Brockmeier D et al.** *Pharmacogenetics,* 2004, vol. 14, 407-417
- **Colombel JF ; Ferrari N ; Debuysere H et al.** *Gastroenterology,* 2000, vol. 118, 1025-1030
- **Schaeffeler E ; Stanulla M ; Greil J ; Schrappe M ; Eichelbaum M ; Zanger UM ; Schwab M.** *Leukemia,* 2003, vol. 17, 1422-1424
- **Hamdan-Khalil R ; Allorge D ; Lo-Guidice JM et al.** *Biochem Biophys Res Commun,* 2003, vol. 309, 1005-1010
- **Lindqvist M ; Haglund S ; Almer S et al.** *Pharmacogenetics,* 2004, vol. 14, 261-265
- **Hamdan-Khalil R ; Gala JL ; Allorge D et al.** TPMT*16 and TPMT*19. *Biochem Pharmacol,* 2005, vol. 69, 525-529
- **Schaeffeler E ; Eichelbaum M ; Reinisch W ; Zanger UM ; Schwab M.** *Hum Mutat,* 2006, vol. 27, 976